# EUROPEAN PATENT SPECIFICATION

(11) **EP 2 946 744 B1**
(45) Date of publication and mention of the grant of the patent: **17.10.2018**
(21) Application number: 15166653.4
(22) Date of filing: 06.05.2015
(51) Int. Cl.: A61C 7/14, A61C 1/08

(54) **IMPLANT POSITIONING DEVICE TO BE FITTED ON TO AN ORTHODONTIC BRACE**
IMPLANTATPOSITIONIERUNGSVORRICHTUNG FÜR EINE ORTHODONTISCHE KLAMMER
DISPOSITIF DE POSITIONNEMENT D'IMPLANT POUR UN APPAREIL ORTHODONTIQUE

(30) Priority: 15.05.2014 TW 103117238
(43) Date of publication of application: 25.11.2015
(73) Proprietor: Densmart Dental Co., Ltd., Taoyuan Hsien 33542 (TW)
(72) Inventor: Yao, Yin Chao, 33542 Taoyuan Hsien (TW)
(74) Representative: Cabinet Chaillot

(56) References cited:
- TW-U- M 398 411
- US-A1- 2009 176 182
- US-A1- 2012 129 123
- Hataichanok Charoenpong ET AL: "A Guide Wire for Miniscrew Placement", O.J. Thai Assoc. Orthod., 1 January 2014 (2014-01-01), pages 1-5, XP055217860, Retrieved from the Internet: URL:http://www.thaiortho.org/journal/wp-co ntent/uploads/journal/current_j4/2014_p1.p df [retrieved on 2015-10-02]
- JANGHYUN PAEK ET AL: "Virtually fabricated guide for placement of the C-tube miniplate", AMERICAN JOURNAL OF ORTHODONTICS AND DENTOFACIAL ORTHOPEDICS, vol. 145, no. 5, 1 May 2014 (2014-05-01), pages 694-702, XP055189923, ISSN: 0889-5406, DOI: 10.1016/j.ajodo.2013.02.036
- KIM ET AL: "Surgical positioning of orthodontic mini-implants with guides fabricated on models replicated with cone-beam computed tomography", AMERICAN JOURNAL OF ORTHODONTICS AND DENTOFACIAL ORTHOPEDICS, MOSBY, ST. LOUIS, MO, US, vol. 131, no. 4, 11 April 2007 (2007-04-11), pages S82-S89, XP022029253, ISSN: 0889-5406, DOI: 10.1016/J.AJODO.2006.01.027 ISBN: 0-9635022-3-9

## Description

### FIELD OF THE INVENTION

The present invention relates generally to an alignment device that is able to determine the position of an orthodontic implant, and more particularly, the present invention relates to a orthodontic implant positioning device that is fitted on to the teeth of a patient that already has at least one existing orthodontic brace.

### BACKGROUND OF THE INVENTION

In the present society, it is very common for a patient to have orthodontic treatment. Generally speaking, the procedure for the correction of both the jaw and teeth of the patient firstly involves an initial visit by the patient to the dental surgeon, in order for the relevant information of the patient to be collected, such as for the growth record of the patient's teeth to be collected, for plaster casts of the patient to be manufactured, for X-ray examination to be done on the interior and the exterior of the oral cavity, and also for photography of the dentition within the patient's mouth to be done, such that the preferred treatment plan for the patient may be developed. As such, the development of a treatment plan for a particular patient is one of the main factors that determines how good the effect is for the orthodontic correction of the jaw and teeth of the patient. In addition, the X-ray examination also plays a very important role in the development of the treatment plan. Moreover, there is still a need for the patient to wear the orthodontic brace for a period of time subsequent to orthodontic correction of their teeth, such that the teeth of the patient may be aligned to the suitable biting position for that particular patient, and mobile orthodontic treatment as well as warranty orthodontic treatment are carried out sequentially in accordance with the treatment plan developed for the patient. At this time, the dental surgeon will also try to examine the status of the teeth of the patient that have undergone orthodontic correction, such that the dental surgeon may be able to judge whether the position of the implant needs to be adjusted, and other implants may need to be inserted.

The orthodontic implant may provide a lot of additional strength. During the process of the orthodontic treatment, when the orthodontist notices phenomenon such as any deviation, any deep bite, or when there are insufficient spaces, the orthodontist may then carry out further adjustments of the orthodontic brace by means of the orthodontic implant, so as to enable the movement of the teeth of the patient to be more accurate, more subtle, more efficient, and as such shortening the duration of the orthodontic treatment of the patient, and also achieving better effect of orthodontic treatment. Prior to the implantation of the orthodontic implant into the oral cavity of the patient, the orthodontist would need to take photographs of the interior of the oral cavity of the patient by means of a photographing system that exists within the oral cavity of the patient. However, the conventional photographing system for the oral cavity of a patient is able to only take X-ray images of the teeth of a patient, so as to enable an initial assessment of the oral cavity of the patient to be carried out. In addition, the conventional oral cavity photographing system does not include an implant positioning device that is able to help the dental surgeon to accurately align the teeth of a patient. Furthermore, the implantation of the orthodontic implant into the oral cavity of the patient may have the risk of injuring the dental roots of the patient, and if there are no supplementary tools to assist during the imaging process, it is thus very difficult for the effect of the orthodontic implant to be confirmed. In addition to this, in situations where an orthodontic brace has already been fitted on to the teeth of a patient, and since the orthodontic brace has already been fitted on to the teeth of the patient, this therefore creates quite a lot of difficulty for the dental surgeon when he or she is confirming the effect of orthodontic treatment.

Document US2009176182 for example discloses an orthodontic implant positioning device adapted to be fixed to an orthodontic brace of a patient and to be used with X-ray machine, the positioning device comprising: a measuring standard member with a radio-opaque material and at least a set of measuring scales and a system to attach it to an orthodontic brace.

The implant positioning device for an orthodontic brace of the present invention is a type of positioning device that enables the positioning of an orthodontic implant without the need to remove the orthodontic brace from the teeth of the patient.

### SUMMARY OF THE INVENTION

The main objective of the present invention is to provide an implant positioning device for patients who already have an orthodontic brace fitted to their teeth, and as such enabling the dental surgeon to be able to obtain information such as the distribution status of the teeth of the patient within the oral cavity, without the need for the existing orthodontic brace of the patient to be removed. Furthermore, the implant positioning device of the present invention also brings great convenience to the dental surgeon, and subsequently enabling the dental surgeon to be able to carry out the alignment and implant surgery.

The other objective of the present invention is that the implant positioning device has a sleeved design, so as to enable the dental surgeon to quickly fit on the implant positioning device on to the orthodontic brace of the patient, or to quickly remove the implant positioning device from the orthodontic brace of the patient. Besides having a simple structure and which is also convenient to use, the implant positioning device of present invention is also able to significantly reduce the detection time required by the dental surgeon.

Yet another objective of the present invention is that the region that is in between the measuring standard member and the encircled member is integrally formed; or, another objective of the present invention is to enable the implant positioning device of the present invention to be exchanged and removed quickly from the orthodontic braces of the patient, either by a direct or an indirect fixing means, so as to bring convenience to the dental surgeon who would need to select a suitable measuring standard member or a encircled member for a number of different patients, on the basis of the different circumstances of oral cavities of the different patients.

The objectives of the present invention are solved by the device as defined in the independent claims 1, 4 and 9.

In order to achieve the aforesaid objectives of the present invention, the implant positioning device for an orthodontic brace of the present invention is fitted on to the orthodontic brace of a patient who has undergone orthodontic treatment. In addition, the implant positioning device for an orthodontic brace of the present invention is used in between an X-ray film and an X-ray projecting machine, and includes: a measuring standard member as well as a encircled member, whereby the detection region is formed from the measuring standard member by means of a radiopaque material. The detection region includes at least one set of measuring scale, and a positioning unit exists on one side of the detection region. The positioning unit may be configured to form a positioning plate, and moreover, the positioning plate is configured to have at least one positioning through hole.

The encircled member forms an encircling main body that may be elastically extended by means of an elastically resilient material. The encircling main body may be hooked on to the outer periphery of two of the orthodontic brace of the patient. The centre of the encircling main body forms a sleeved space, and whereby the size of the sleeved space may be changed. Moreover, the orthodontic brace of the patient is to be fitted in the sleeved space of the encircling main body. In addition to this, the encircling main body may also include a connecting unit, and the connecting unit may be configured to correspond to the stop block of the positioning through hole, and a fixing means that may be grouped or removed repeatedly is formed collectively by the connecting unit as well as the positioning unit.

In accordance with an embodiment of the present invention, the implant positioning device for an orthodontic brace of the present invention includes: a measuring standard member, a fixing member and a plurality of encircled members, whereby the measuring standard member forms a detection region by means of a radiopaque material, and the detection region may include at least one set of measuring scale. A positioning unit is to be found on one side of the detection region; the positioning unit may be configured to be a positioning plate, and the positioning plate may be configured to have at least one positioning through hole.

The fixing member includes a connecting unit that is assembled and which corresponds to the positioning unit. Furthermore, one side of the surface of the fixing member may include at least one slot portion that corresponds to the orthodontic brace of the patient. The connecting unit may be configured as a stop block that corresponds to the two positioning through holes. Also, a fixing means that may be grouped or removed repeatedly may be formed collectively by the connecting unit as well as the positioning unit.

The plurality of encircled members are respectively in connection with the two sides of the fixing member. The encircling main body that may be elastically extended is made up of an elastically resilient material; and the centre of the encircling main body forms a sleeved space, whereby the size of the sleeved space may be changed. Moreover, the orthodontic brace of the patient is to be fitted in the sleeved space of the encircling main body. The encircling main body may be sleeved on to the outer periphery of a single orthodontic unit of the orthodontic brace of the patient.

In accordance with a preferred exemplary use of the present invention, the implant positioning device for an orthodontic brace is to be fitted on to the orthodontic brace of a patient who has undergone orthodontic treatment, and the implant positioning device for an orthodontic brace of the present invention is used in between an X-ray film and an X-ray projecting machine, and includes a measuring standard member. The measuring standard member forms a detection region, by means of a radiopaque material. In addition, the detection region includes at least one set of measuring scale. A encircled member, which is in connection with the encircling main body of the orthodontic brace of a patient, by means of one side of the measuring standard member integrally extending and being in connection with the encircling main body of the orthodontic brace. Furthermore, a sleeved space is formed collectively by the encircling main body as well as the measuring standard member.

In accordance with a preferred exemplary use of the present invention, at least one orthodontic unit of the orthodontic brace of the patient may pass through the sleeved space of the encircling main body in order for the orthodontic brace to be fixed.

The encircling main body may be fixed in the slot in the center of at least one orthodontic unit of the orthodontic brace of the patient.

The benefits of the implant positioning device for an orthodontic brace of the present invention is that the measuring standard member that forms the measuring scale may be in direct connection or an indirect connection with the encircled member that may be extended, and as such forming an implant positioning device that may be conveniently grouped on to or removed from the orthodontic brace of a patient. This may also enable different patients who require orthodontic treatment and also with different dental status to be able to directly have examination of their teeth without the need to remove their existing orthodontic brace. Moreover, under the circumstances of repeated grouping as well as removal, the implant positioning device for orthodontic brace of the present invention may thus enable the dental surgeon to correctly determine the position of the implant surgery by means of the measuring standard member.

In addition to the above, the measuring standard member and the encircled member may be integrally formed; or, the measuring standard member may be in a direct connection or an indirect connection with the encircled member by means of a fixing means, and this may bring convenience to the dental surgeon, since the dental surgeon may be able to quickly group the implant positioning device on to the orthodontic brace of a patient, and that when finished using, the dental surgeon may be able to quickly remove the implant positioning device from the orthodontic brace of the patient. Moreover, the dental surgeon is able to accurately determine the position of implantation of the implant positioning device in the oral cavity of the patient. Furthermore, the implant positioning device for an orthodontic brace of the present invention may be used by any patient who requires orthodontic treatment, and as such also increasing the efficiency of diagnosis and treatment by the dental surgeons.

### BRIEF DESCRIPTION OF THE DRAWINGS

The present invention can be understood in more detail by reading the subsequent detailed description in conjunction with the examples and preferred exemplary embodiments made to the accompanying drawings, wherein:
Fig. 1 is an exploded view of the implant positioning device of the present invention in accordance with a first preferred exemplary embodiment of the present invention.
Fig. 2 is a schematic diagram showing that the implant positioning device of Fig. 1 has been fitted on the orthodontic brace of a patient in accordance with the present invention.
Fig. 3 is a schematic diagram showing the implant positioning device of Fig. 1 that is in use, and is being used together with the X-ray imaging device to for taking photograph of a dental root in accordance with the present invention.
Fig. 4 is a schematic diagram showing an image that has been projected directly on an X-ray film by means of the implant positioning device in accordance with the present invention.
Fig. 5 is another schematic diagram of the implant positioning device of the present invention that is in use, and is being used together with the X-ray imaging device to take photograph of a dental root in accordance with the present invention.
Fig. 6 is an exploded view of the implant positioning device of the present invention in accordance with a second preferred exemplary embodiment of the present invention.
Fig. 7 is a schematic diagram showing the implant positioning device of Fig. 6 that has been fitted on an orthodontic brace of a patient in accordance with the present invention.
Fig. 8 is a schematic diagram showing the implant positioning device of Fig. 6 is in use, and is being used together with an X-ray imaging device to take photograph of a dental root in accordance with the present invention.
Fig. 9 is a schematic diagram showing that the implant positioning device of Fig. 6 is in use, and is being used together with an X-ray imaging device to take photograph of another dental root in accordance with the present invention.
Fig. 10 is a structural diagram showing the implant positioning device in accordance with a third preferred exemplary embodiment of the present invention.
Fig. 11 is a schematic diagram showing the implant positioning device of Fig. 10 that has been fitted on to an orthodontic brace of a patient in accordance with the present invention.
Fig. 12 is another schematic diagram showing the implant positioning device of Fig. 10 that has been fitted on to an orthodontic brace of a patient in accordance with the present invention.

### DETAILED DESCRIPTION OF THE PREFERRED EMBODIMENT

The accompanying drawings are included to provide a further understanding of the invention, and are incorporated in and constitute a part of this specification. The drawings illustrate the preferred exemplary embodiments of the invention and, together with the description, serve to explain the principles of the invention.

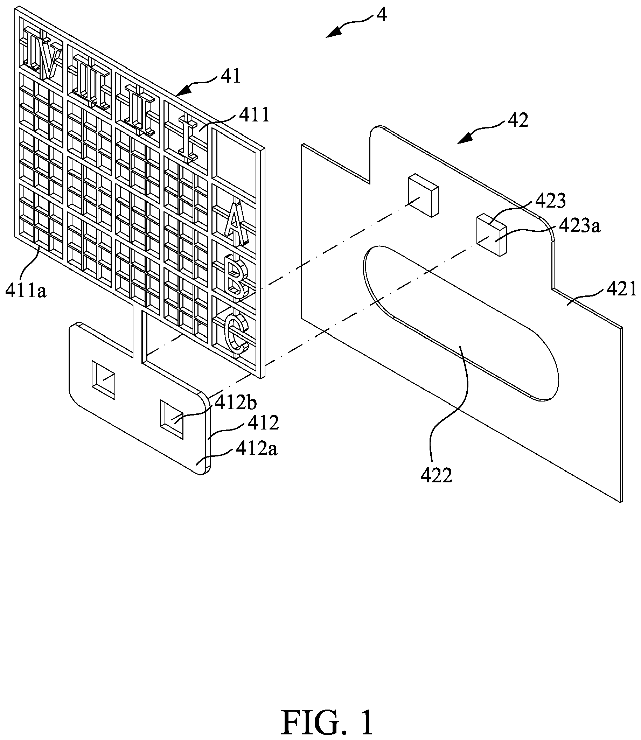

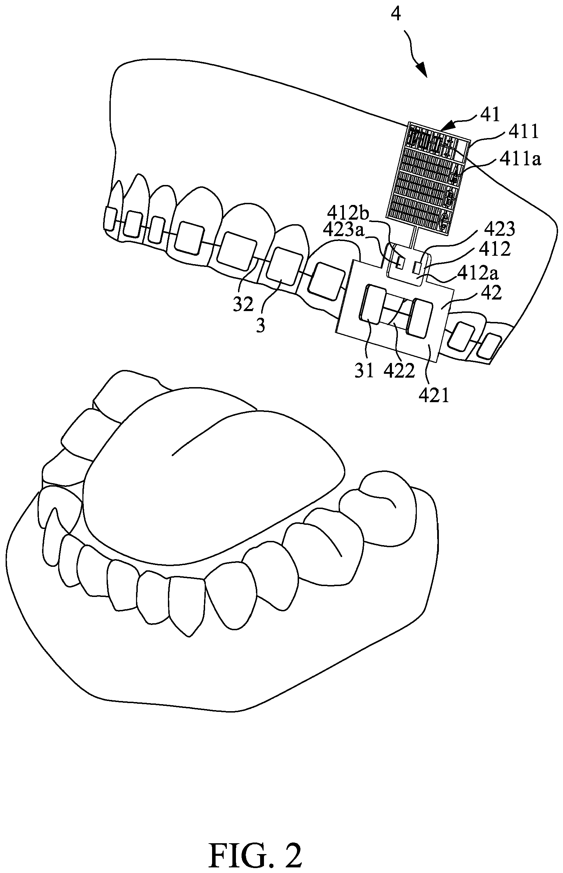

As shown in Fig. 1 and Fig. 2, in accordance with a first preferred exemplary embodiment of the present invention, the implant positioning device 4 of the present invention includes a measuring standard member 41 and a encircled member 42. The measuring standard member 41 may form a detection region 411 by means of a radiopaque material. The detection region 411 may be provided with at least one set of measuring scale 411a. The at least one set of measuring scale 411a may be formed by means of the plurality of horizontal lines and the plurality of vertical lines that are interlaced. The at least one set of measuring scale 441a may have identification symbols, such as A, B, C, I, II and III, as shown in Fig. 1 and Fig. 2. One side of the detection region 411 may have a positioning unit 412 that is extended from the detection region 411. The positioning unit 412 may be a positioning plate 412a that is a rectangular shape. In addition, the surface of the positioning plate 412a may include a plurality of positioning through holes 412b.

Also, as shown in Fig. 1 and Fig. 2, the encircled member 42 of the present invention may form an encircling main body 421 by means of an elastically resilient material. The encircling main body 421 may be formed as a rectangular body that may be elastic. Moreover, the central region of the encircling main body 421 may include a sleeved space 422, and the size of the sleeved space 422 may be changed. The sleeved space 422 may be formed as a geometrical through hole. The sleeved space 422 of which the size may be elastically changed may be used to rapidly encircle two orthodontic units 31. In other words, the sleeved space 422 may be suitable for the oral conditions of different patients. The encircling main body 421 may encircle the outer peripheries of two orthodontic units 31 of the orthodontic brace 3. The encircling main body 421 may further include a connecting unit 423 on one side of the encircling main body 421. The connecting unit 423 may include at least one stop block 423a. The at least one stop block 423a may be fixed on to the corresponding geometrical shape of the positioning through hole 412b.

However, a fixing means between the positioning unit 412 of the measuring standard member 41 and the connecting unit 423 of the encircled member 42 is only an exemplary embodiment, but should not be limited to the exemplary embodiment set forth herein. In other words, the fixing means that is in between the positioning unit 412 and the connecting unit 423 that are engaged with each other may be one of an adhesive element, a retaining element, a fastening element and a holding element.

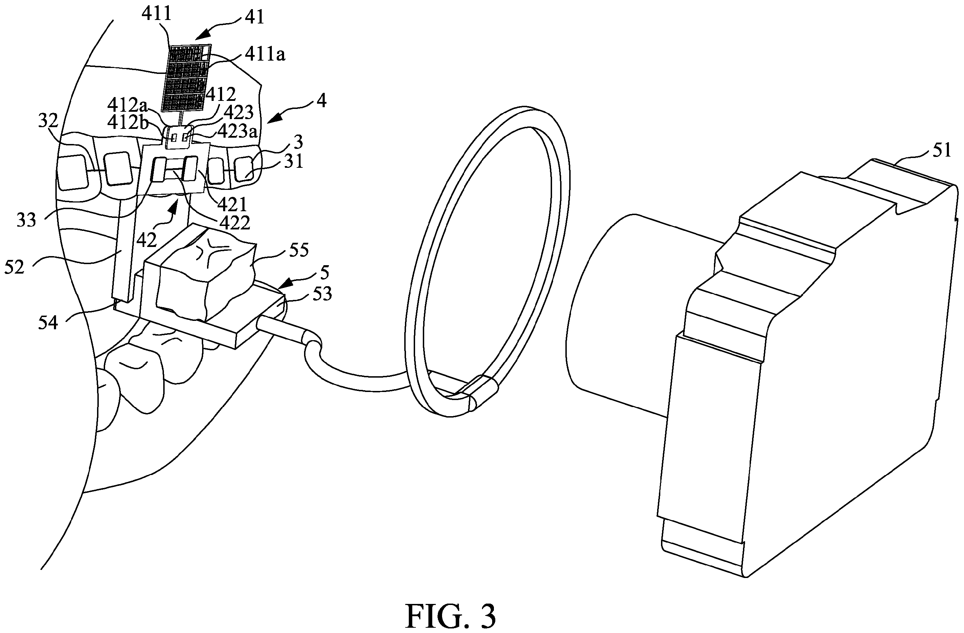

As shown in Fig. 3, when the implant positioning device 4 of the present invention is used, the sleeved space 422 of the encircled member 42 may encircle two orthodontic units 31 of the orthodontic brace 3. In other words, the encircling main body 421 of the encircled member 42 may encircle a concave groove 33 that is formed by the outer peripheries of the orthodontic units 31. Subsequent to this, the positioning through hole 412b of the positioning unit 412 of the measuring standard member 41 and the at least one stop blocks 423a of the connecting unit 423 of the encircled member 42 may be engaged with each other. Accordingly, the implant positioning device 4 of the present invention is completely configured.

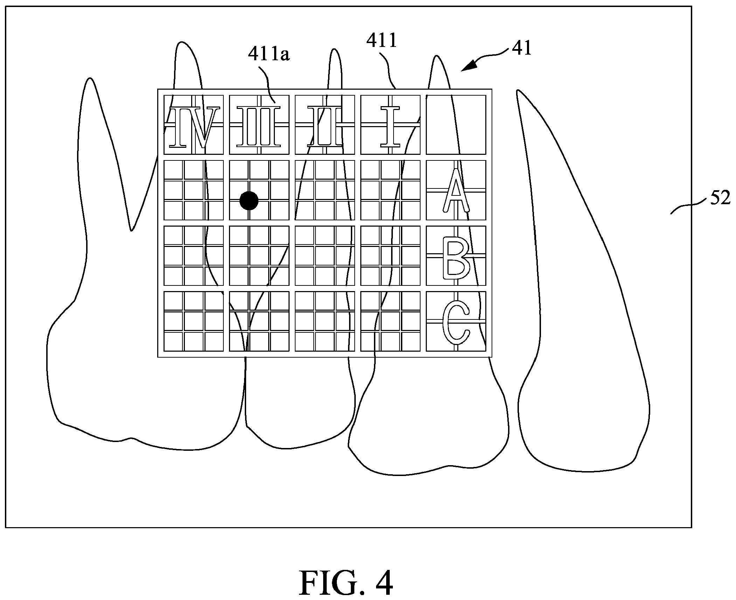

Please continue to refer to Fig. 3, when the present invention is used in practice, an imaging device 5 may also be used in the present invention. The imaging device 5 may include an X-ray projecting machine 51, an X-ray film 52, a base 53, an engaging section 54 and a tooth mold material 55. In accordance with a preferred exemplary embodiment, the X-ray film 52 that is engaged with the engaging section 54 of the base 53 may be used together with the implant positioning device 4 of the present invention. The tooth mold material 55 may be provided for a dental bite impression to be made on the base 53. After the dental bite impression is formed on the tooth mold material 55, the measurement scale 411a of the measuring standard member 41, the X-ray projecting machine 51 and the X-ray film 52 are all adjusted in parallel. When the image of the teeth of a patient is projected on the X-ray film 52 by the X-ray projecting machine 51, the X-ray film 52 may show the positioning relationship between the teeth of a patient, and the measurement scale 411a of the measuring standard member 41 may be shown on the X-ray film 52, as shown in Fig. 4. As such, a dental surgeon may determine where the implant position of a tooth in the implant region is marked on the basis of the image shown in the X-ray film 52, so as to position a tooth implant in the implant position.

In accordance with another preferred exemplary embodiment of the present invention, the X-ray film 52 may not be fixed by means of the engaging section 54 of the base 53, but the X-ray film 52 may be directly fixed within a region of the oral cavity of the patient that is photographed, by means of the fingers of a patient. On the other hand, the X-ray film 52 may be directly fixed within the oral cavity of a patient by means of the teeth engagement of a patient. In addition, the X-ray film 52 may be installed on the implant positioning device 4 such that the X-ray film 52 may provide an image of teeth of a patient and the measuring scale 441a of the measuring standard member 41 may be provided.

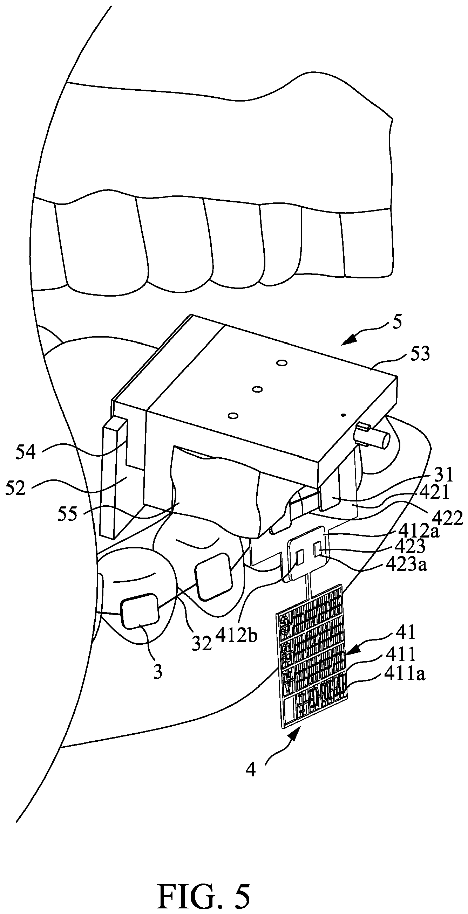

As shown in Fig. 5, when the lower row of teeth of a patient is required to be irradiated, the plurality of encircled members 42 that are turned upside down may encircle the orthodontic brace 3 on the lower row of teeth in accordance with the first preferred exemplary embodiment of the present invention. In addition, the imaging device 5 may also be turned upside down. In accordance with the aforesaid method, the positioning unit 412 of the measuring standard member 41 may be sequentially engaged with the connecting unit 423 of the plurality of encircled members 42. The X-ray film 52 may be placed upside down on the engaging section 54 of the base 53. After an image of the teeth of a patient is projected on the X-ray film 52 by the X-ray projecting machine 51, the status of the teeth may be rapidly and accurately determined by a dental surgeon.

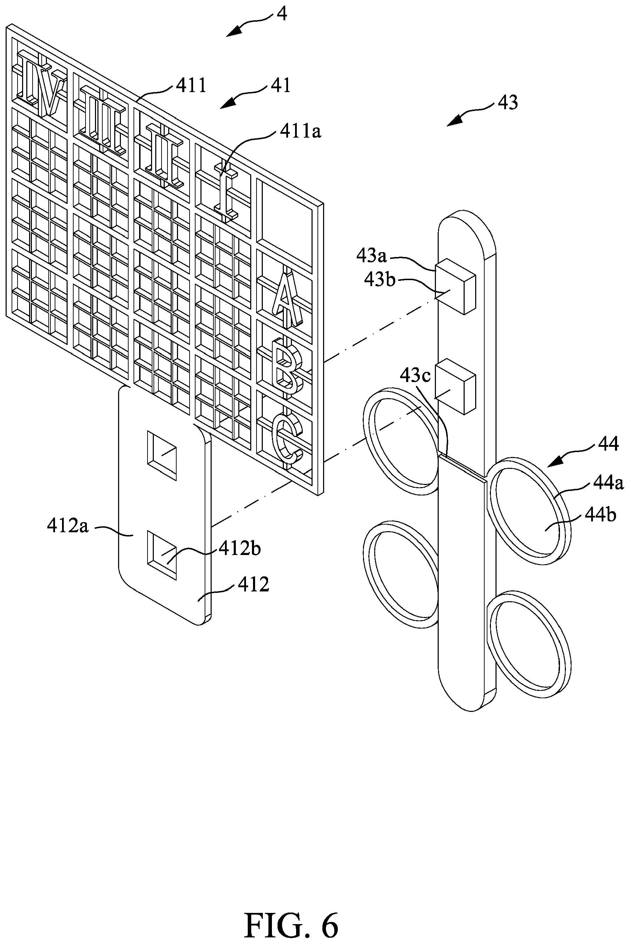

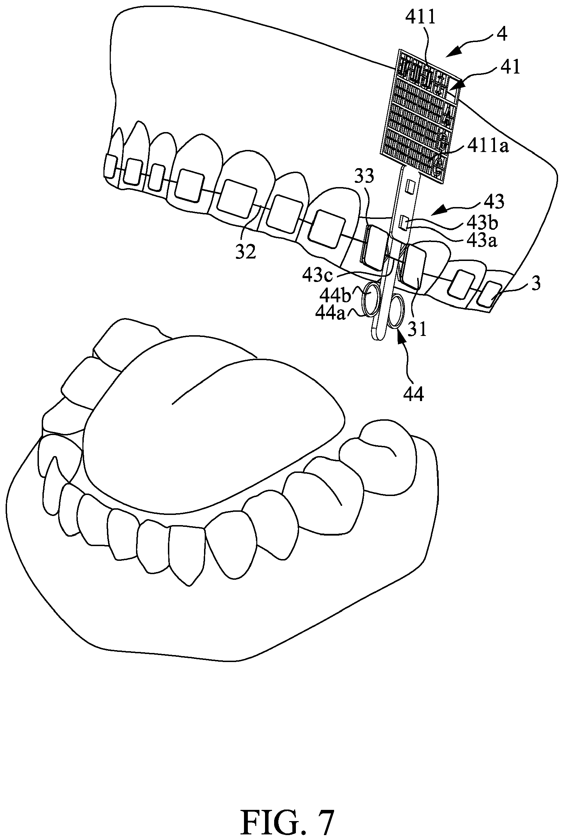

In addition, as shown in Fig. 6 and Fig. 7, in accordance with the second preferred exemplary embodiment of the present invention, the implant positioning device 4 of the present invention may include a measuring standard member 41, a fixing member 43 and a plurality of encircled members 44. It should be noted that the structure of the measuring standard member 41 of the second preferred exemplary embodiment is the same as the structure of the measuring standard member 41 of the first preferred exemplary embodiment, such that the measuring standard member 41 of the second preferred exemplary is not repeated herein.

Also, as shown in Fig. 6 and Fig. 7, the fixing member 43 may include a connecting unit 43a. The connecting unit 43a may include at least one stop block 43b that corresponds to the positioning through hole 412b of the measuring standard member 41. A fixing means that can be reassembled or unattached may be collectively formed by the at least one stop block 43b and the positioning through hole 412b. At least one slot portion 43c may be formed on a side surface of the fixing member 43. The structure of the at least one slot portion 43c may enable the orthodontic metal wire 32 of the orthodontic brace 3 to be fitted for the at least one slot portion 43c.

Similarly, the fixing means between the connecting unit 43a and the positioning through hole 412b may be an example, for the convenience of illustration only. In accordance with other preferred exemplary embodiments, the fixing means between the positioning unit 412 and the connecting unit 43a that are engaged with each other may be one of an adhesive element, a retaining element, a fastening element and a holding element.

Please continue to refer to Fig. 6 and Fig. 7, the plurality of encircled members 44 may be arranged in parallel and may also be arranged symmetrically with respect to the two opposite sides of the fixing member 43. The plurality of encircled members 44 may form an encircling main body 44a. The encircling main body 44a may be elastically extended by means of an elastically resilient material. The size of a sleeved space 44b may be formed and changed by encircling the center of the encircling main body 44a. The encircling main body 44a of the two encircled members of the plurality of encircled members 44 may be used to encircle the outer peripheries of two adjacent orthodontic units of the orthodontic brace 3. As such, the orthodontic brace 3 may be fitted on to and housed in the sleeved space 44b of the encircling main body 44a.

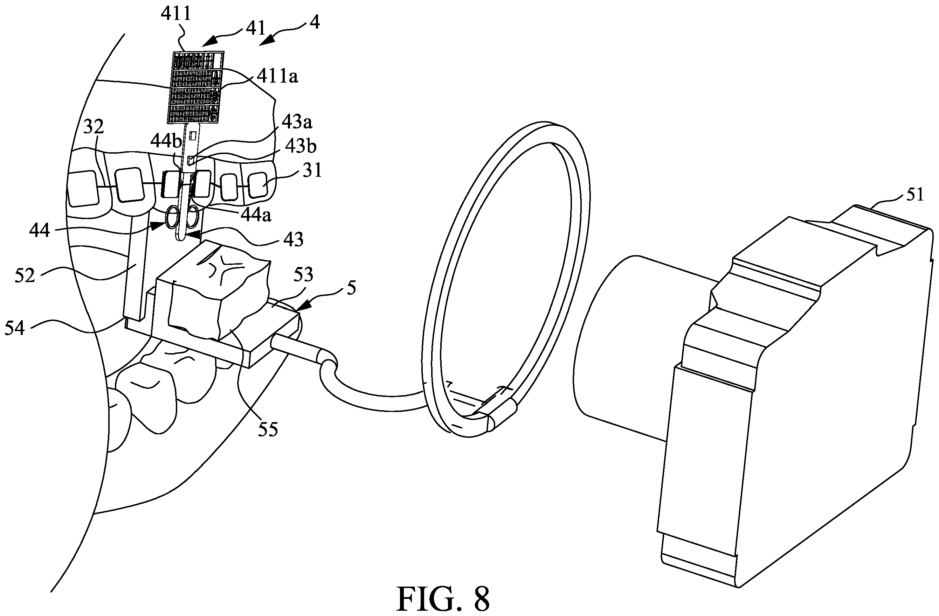

As shown in Fig. 8, in accordance with the second preferred exemplary embodiment, when the implant positioning device 4 is being used, the fixing member 43 may be aligned with the teeth regional position in which the implant is inserted, and the orthodontic metal wire 32 of the orthodontic brace 3 may be detached from the teeth of the patient. As such, the plurality of encircled members 44 on the two sides of the fixing member may encircle the orthodontic units 31 of the orthodontic brace 3, respectively. In other words, the size of the sleeved space 44b may be changed due to the elastic deformation of the plurality of encircled members 44, such that the outer peripheries of the orthodontic units 31 of the orthodontic brace 3 may be encircled inside the sleeved space 44b. When the present invention is being used, the sleeved space 44b of the plurality of encircled members 44 may encircle a orthodontic unit 31 of the orthodontic brace 3, and following this, the sleeved space 44b on the other side of the plurality of encircled members 44 may encircle the other side of the orthodontic unit 31 in accordance with the same method. Possibly, the plurality of encircled members 44 may encircle the plurality of orthodontic units 31 at the same time. After that, the positioning unit 412 of the measuring standard member 41 and the at least one slot portion 43c of the fixing member 43 may be engaged with each other. Finally, the orthodontic metal wire 32 that can be removed and the at least one slot portion 43c of the fixing member 43 may be assembled and engaged with each other on the orthodontic brace 3. Accordingly, the implant positioning device 4 of the present invention is completely configured. The other steps of the second preferred exemplary embodiment of the present invention is the same as the steps of the first preferred exemplary embodiment of the present invention.

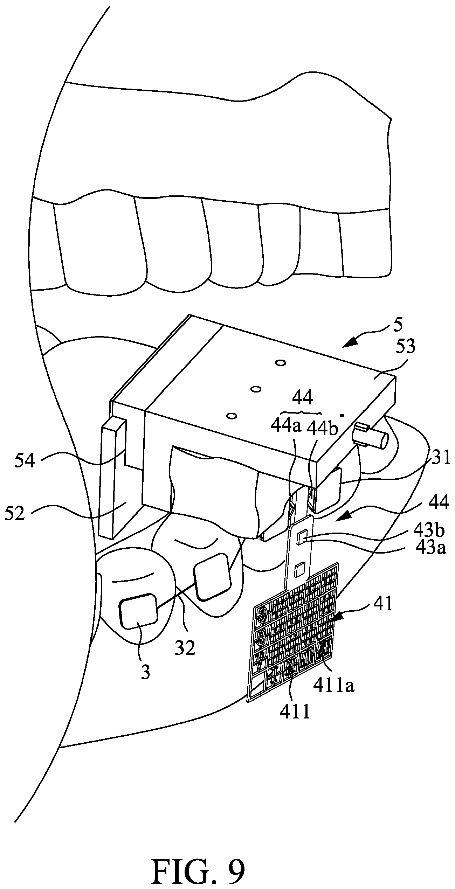

In accordance with another preferred exemplary embodiment of the present invention, as shown in Fig. 9, when the lower row of teeth of a patient is required to be irradiated by means of the implant positioning device 4 of the present invention, the fixing member 43 that is turned upside down may enable the encircled member 44 to encircle the orthodontic brace 3 on the lower row of teeth of a patient. Subsequent to this, the measuring standard member 41 may be placed upside down so as to enable the positioning through hole 412b and the connecting unit 43a of the fixing member 43 to be assembled and to be engaged with each other. The other steps of the preferred exemplary embodiment of the present invention are the same as the steps of the first preferred exemplary embodiment of the present invention, and are therefore not repeated herein.

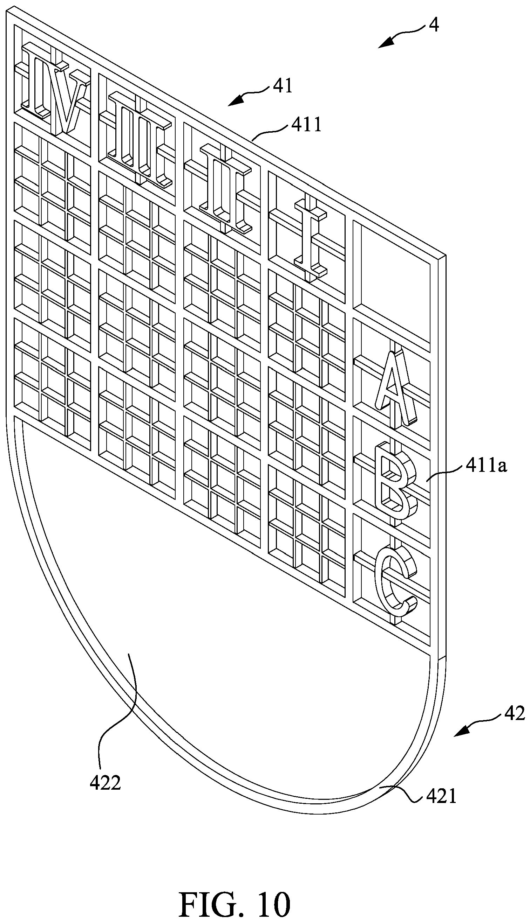

In accordance with the third preferred exemplary embodiment of the present invention, as shown in Fig. 10, the present invention further provides an implant positioning device 4 for use as an orthodontic brace 3. The implant positioning device 4 may include a measuring standard member 41. The measuring standard member 41 may form a detection region 411 by means of a radiopaque material, and the detecting region 411 may include at least one set of measuring scale 411a. Moreover, the implant positioning device 4 may also include a encircled member 42. The encircled member 42 may be integrated with one side of the measuring standard member 41 so as to form an encircling main body 421 of the orthodontic brace 3. Moreover, a sleeved space 422 may be collectively formed by the encircling main body 421 and the measuring standard member 41.

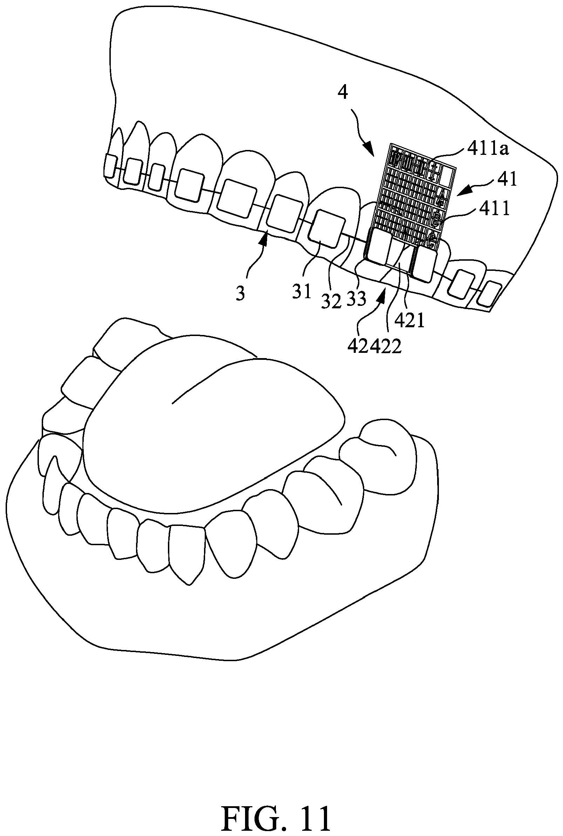

As shown in Fig. 11, in accordance with the third preferred exemplary embodiment of the present invention, when the implant positioning device 4 of the orthodontic brace 3 of the present invention is used, the outer periphery of at least one orthodontic unit 31 of the orthodontic brace 3 may be encircled by the concave groove 33 of the encircling main body 421, such that the at least one orthodontic unit 31 of the orthodontic brace 3 may be fixed inside the sleeved space 422 of the encircling main body 421. As such, the measuring standard member 41 is placed on the gum of the patient, so as to enable the X-ray projection to be performed. Therefore, the position of a tooth implant inside the oral cavity of a patient may be accurately determined. The other steps of the third preferred exemplary embodiment of the present invention are the same as the steps of the second preferred exemplary embodiment of the present invention, and are thus not repeated herein.

Also referring to Fig. 11, Fig. 11 also shows that the implant positioning device 4 of the present invention is used to determine the usage of the teeth on the upper jaw of the patient. However, when the teeth on the lower jaw of the patient is required to be determined, the measuring standard member 41 and the fixing member 42 may only be placed upside down, so as to enable the measuring standard member 41 to be placed on the teeth of the lower jaw and gum of the patient. As such, the X-ray projection may be performed such that the position of a teeth implant may be accurately obtained.

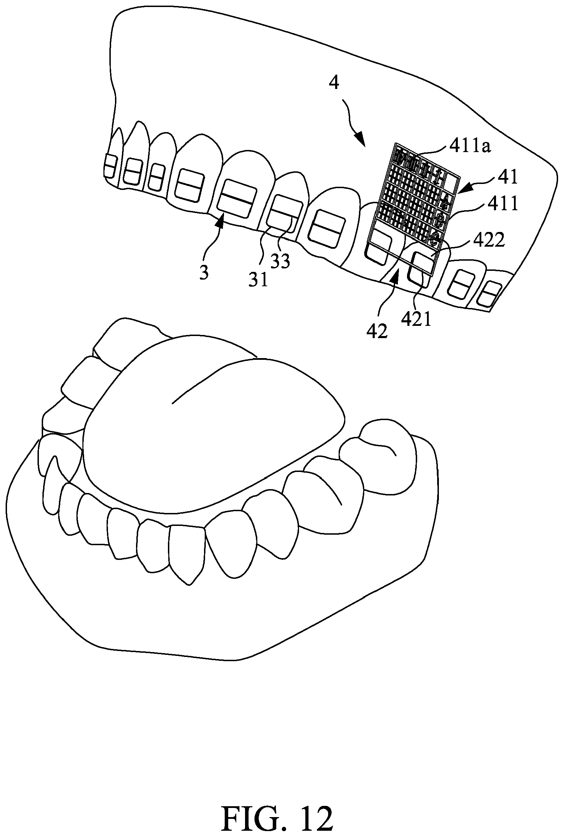

A shown in Fig. 12, in accordance with another method of the third preferred exemplary embodiment of the present invention, when the orthodontic metal wire (not shown) of the orthodontic brace 3 is removed, the concave grooves 33 of the plurality of orthodontic units 31 of the orthodontic brace 3 may be exposed. Subsequent to this, the encircling main body 421 may be fixed in the concave groove 33 of at least one orthodontic unit 31 of the orthodontic brace 3, such that some areas of the at least one orthodontic unit 31 of the orthodontic brace 3 may be inside the sleeved space 422 of the encircling main body 421. When the orthodontic metal wire (not shown) is re-installed on the orthodontic brace 3 and the measuring standard member 41 and the encircled member 42 are fixed, the X-ray projection may be performed. Since the other steps of the third preferred exemplary embodiment of the present invention are the same as the steps of the first and the second preferred exemplary embodiments of the present invention, the other steps of the third preferred exemplary embodiment of the present invention are thus not repeated herein. In summary, the benefit of the present invention is to enable the patients having different teeth statuses to not need to remove their orthodontic brace 3, and that an X-ray projection may be performed by means of different sizes of the sleeved space of the encircled member encircling at least one orthodontic unit of the orthodontic brace 3. In other words, the technical means of the encircled member including a measuring standard member with measuring scale may enable a dental surgeon to obtain the required information about the orthodontic correction and the teeth implant on the basis of the measuring scale. In addition, the implant positioning device of the present invention may be re-installed and may still be positioned accurately. Therefore, in accordance with the implant positioning device of the present invention, the correction situation and the position of the implant surgery may be accurately determined by a dental surgeon. Accordingly, accuracy and efficiency of diagnosis and treatment by the dental surgeon may be significantly improved based on the implant positioning device of the present invention.

Although the preferred exemplary embodiments of the present invention have been described with reference to the preferred exemplary embodiments thereof, it should be understood that the scope of the present invention is defined by the appended claims.

## Claims

1. An orthodontic implant positioning device adapted to be fixed to an orthodontic brace (3) of a patient and used in between an X-ray projecting machine (51) and an X-ray film (52), the implant positioning device comprising:
a measuring standard member (41), forming a detection region (411) by means of a radiopaque material and having a positioning unit (412) on one side of the detection region (411), wherein the detection region (411) is provided with at least one set of measuring scales (411a); and
at least one encircled member (42), forming an encircling main body (421) made up of an elastically resilient material, wherein the central region of the encircling main body (421) includes a sleeved space (422), wherein the size of the sleeved space (422) is elastically changed when the orthodontic brace (3) is fitted and housed in the sleeved space (422) of the encircling main body (421), the encircling main body (421) further comprises a connecting unit (423), wherein a fixing means is provided between the positioning unit (412) and the connecting unit (423) so that said positioning unit (412) and said connecting unit (423) are engageable with each other.

2. The orthodontic implant positioning device in accordance to claim 1, wherein the encircling main body (421) is used to encircle the outer periphery of two adjacent orthodontic units (31) of the orthodontic brace (3).

3. The orthodontic implant positioning device in accordance to claim 1, wherein the positioning unit (412) comprises a positioning plate (412a), the positioning plate (412a) comprises at least one positioning through hole (412b), and the connecting unit (423) comprises at least one stop block (423a) that corresponds to the at least one positioning through hole (412b).

4. An orthodontic implant positioning device adapted to be fixed to an orthodontic brace (3) of a patient and used in between an X-ray projecting machine (51) and an X-ray film (52), the implant positioning device comprising:
a measuring standard member (41), forming a detection region (411) by means of a radiopaque material and having a positioning unit (412) on one side of the detection region (411), wherein the detection region (411) is provided with at least one set of measuring scales (411a);
a fixing member (43), having a connecting unit (43a), wherein a fixing means is provided between the positioning unit (412) and the connecting unit (43a) so that said positioning unit (412) and said connecting unit (423) are engageable with each other; and
a plurality of encircled members (44), respectively connected to the two sides of the fixing member (43), and forming an encircling main body (44a) made up of an elastically resilient material, wherein the size of a sleeved space (44b) of each encircled member (44) is changeable due to the elastic deformation of the encircled member (44), when the orthodontic brace (3) is fitted and housed in the sleeved space (44b) of the encircling main body (44a).

5. The orthodontic implant positioning device in accordance to claim 4, wherein at least one slot portion (43c) is formed on a surface of and at one side of the fixing member (43).

6. The orthodontic implant positioning device in accordance to claim 4, wherein the plurality of encircled members (44) are arranged in parallel and are arranged symmetrically with respect to the two opposite sides of the fixing member (43).

7. The orthodontic implant positioning device in accordance to claim 6, wherein two encircling main bodies of the plurality of the encircled members (44) is used to encircle the outer peripheries of two adjacent orthodontic units (31) of the orthodontic brace (3), respectively.

8. The orthodontic implant positioning device in accordance to claim 4, wherein the positioning unit (412) comprises a positioning plate (412a), the positioning plate (412a) comprises at least one positioning through hole (412b), and the connecting unit (43a) comprises the plurality of stop blocks (43b) that correspond to the plurality of positioning through holes (412b).

9. An orthodontic implant positioning device adapted to be fixed to an orthodontic brace (3) of a patient and used in between an X-ray projecting machine (51) and an X-ray film (52), the implant positioning device comprising:
a measuring standard member (41), forming a detection region (411) by means of a radiopaque material and having a positioning unit (412) on one side of the detection region (411), wherein the detection region (411) is provided with at least one set of measuring scales (411a); and
a encircled member (42), integrated with one side of the measuring standard member and forming an encircling main body (421) that is connectable to the orthodontic brace (3), wherein a sleeved space (422) is collectively formed by the encircling main body (421) and the measuring standard member (41), wherein the encircling main body is elastically extended when the orthodontic brace (3) is fitted and housed in the sleeved space (422) of the encircling main body (421).

10. The implant positioning device for orthodontic brace in accordance to claim 9, wherein at least one orthodontic unit of the orthodontic brace (3) is fixed in the sleeved space (422) of the encircling main body (421).

11. The implant positioning device for orthodontic brace in accordance to claim 9, wherein the encircling main body (421) is fixed in a central groove of at least one orthodontic unit of the orthodontic brace (3).

## Patentansprüche

1. Orthodontische Implantatpositionierungsvorrichtung zur Befestigung an einer orthodontischen Klammer (3) eines Patienten, zur Verwendung zwischen einem Röntgengerät (51) und einem Röntgenfilm (52), wobei die Implantatpositionierungsvorrichtung folgendes umfasst:
ein Standard-Messglied (41), das einen Erfassungsbereich (411) bildet, mittels eines röntgendichten Materials, mit einer Positionierungseinheit (412) an einer Seite des Erfassungsbereichs (411), wobei der Erfassungsbereich (411) mindestens einen Messskalasatz (411a) umfasst; und
mindestens ein umschlossenes Teil (42), der einen umschließenden Hauptkörper (421) bildet, aus einem federnd elastischen Material, wobei der mittlere Bereich des umschließenden Hauptkörpers (421) einen umhüllten Raum (422) umfasst, wobei die Größe des umhüllten Raums (422) sich elastisch ändert, wenn die orthodontische Klammer (3) eingesetzt und in dem umhüllten Raum (422) des umschließenden Hauptkörpers (421) untergebracht ist,
der umschließende Hauptkörper (421) umfasst des weiteren eine Verbindungseinheit (423), wobei ein Befestigungsmittel zwischen der Positionierungseinheit (412) und der Verbindungseinheit (423) vorgesehen ist, so dass besagte Positionierungseinheit (412) und besagte Verbindungseinheit (423) ineinander einkuppelbar sind.

2. Orthodontische Implantatpositionierungsvorrichtung nach Anspruch 1, wobei der umschließende Hauptkörper (421) verwendet wird, um den Außenumfang von zwei benachbarten orthodontischen Einheiten (31) der orthodontischen Klammer (3) zu umgeben.

3. Orthodontische Implantatpositionierungsvorrichtung nach Anspruch 1, wobei die Positionierungseinheit (412) eine Positionierungsplatte (412a) umfasst, die Positionierungsplatte (412a) umfasst mindestens eine Positionierungs-Durchgangsbohrung (412b), und die Verbindungseinheit (423) umfasst mindestens einen Anschlagblock (423a) der der mindestens einen Positionierungs-Durchgangsbohrung (412b) entspricht.

4. Orthodontische Implantatpositionierungsvorrichtung zur Befestigung an einer orthodontischen Klammer (3) eines Patienten, zur verwendung zwischen einem Röntgengerät (51) und einem Röntgenfilm (52), wobei die Implantatpositionierungsvorrichtung folgendes umfasst:
ein Standard-Messglied (41), das einen Erfassungsbereich (411) bildet, mittels eines röntgendichten Materials, mit einer Positionierungseinheit (412) an einer Seite des Erfassungsbereichs (411), wobei der Erfassungsbereich (411) mindestens einen Messskalasatz (411a) umfasst;
ein Befestigungselement (43), mit einer Verbindungseinheit (43a), wobei ein Befestigungsmittel zwischen der Positionierungseinheit (412) und der Verbindungseinheit (43a) vorgesehen ist, so dass besagte Positionierungseinheit (412) und besagte Verbindungseinheit (423) ineinander einkuppelbar sind; und
eine Vielzahl von umschlossenen Teilen (44), die jeweils mit den beiden Seiten des Befestigungsteils (43) verbunden sind und einen umschließenden Hauptkörper (44a) bilden, aus einem federnd elastischen Material, wobei die Größe eines umhüllten Raums (44b) jedes umschlossenen Teils (44) veränderbar ist, wegen der elastischen Verformung des umschlossenen Teils (44), wenn die orthodontische Klammer (3) eingepasst und in dem umhüllten Raum (44b) des umschließenden Hauptkörpers (44a) untergebracht ist.

5. Orthodontische Implantatpositionierungsvorrichtung nach Anspruch 4, wobei mindestens ein Schlitzteil (43c) auf einer Oberfläche des Befestigungsteils (43) und an einer Seite davon gebildet wird.

6. Orthodontische Implantatpositionierungsvorrichtung nach Anspruch 4, wobei die Vielzahl von umschlossenen Teilen (44) parallel und symmetrisch in Bezug auf die beiden gegenüberliegenden Seiten des Befestigungsteils (43) angeordnet sind.

7. Orthodontische Implantatpositionierungsvorrichtung nach Anspruch 6, wobei zwei umschließende Hauptkörper aus der Vielzahl der umschlossenen Teile (44) verwendet werden, um die Außenumfänge von zwei benachbarten orthodontischen Einheiten (31) der orthodontischen Klammer (3) zu umschließen.

8. Orthodontische Implantatpositionierungsvorrichtung nach Anspruch 4, wobei die Positionierungseinheit (412) eine Positionierungsplatte (412a) umfasst, die Positionierungsplatte (412a) umfasst mindestens eine Positionierungs-Durchgangsbohrung (412b), und die Verbindungseinheit (43a) umfasst diejenige Vielzahl von Anschlagblöcken (43b), die der Vielzahl von (412b) Positionierungs-Durchgangsbohrungen entspricht.

9. Orthodontische Implantatpositionierungsvorrichtung zur Befestigung an einer orthodontischen Klammer (3) eines Patienten, zur Verwendung zwischen einem Röntgengerät (51) und einem Röntgenfilm (52), wobei die Implantatpositionierungsvorrichtung folgendes umfasst:
ein Standard-Messglied (41), das einen Erfassungsbereich (411) bildet, mittels eines röntgendichten Materials, mit einer Positionierungseinheit (412) an einer Seite des Erfassungsbereichs (411), wobei der Erfassungsbereich (411) mindestens einen Messskalasatz (411a) umfasst; und
ein umschlossenes Teil (42), das mit einer Seite des Standard-Messglieds integriert ist und einen umschließenden Hauptkörper (421) bildet, der mit der orthodontischen Klammer (3) verbindbar ist, wobei ein umhüllter Raum (422) gemeinsam von dem umschließenden Hauptkörper (421) und dem Standard-Messglied (41) gebildet wird, wobei die umschließende Hauptkörper federnd ausgedehnt wird, wenn die orthodontische Klammer (3) eingepasst ist und in dem umhüllten Raum (422) des umschließenden Hauptkörpers (421) untergebracht ist.

10. Implantatpositionierungsvorrichtung für orthodontische Klammer nach Anspruch 9, wobei mindestens eine orthodontische Einheit der orthodontischen Klammer (3) in dem umhüllten Raum (422) des umschließenden Hauptkörpers (421) befestigt ist.

11. Implantatpositionierungsvorrichtung für orthodontische Klammer nach Anspruch 9, wobei der umschließende Hauptkörper (421) in einer mittleren Rille mindestens einer orthodontischen Einheit der orthodontischen Klammer (3) befestigt ist.

## Revendications

1. Dispositif de positionnement d'implant orthodontique adapté pour être fixé à un appareil orthodontique (3) d'un patient et utilisé entre une machine de projection de rayons X (51) et un film radiologique (52), le dispositif de positionnement d'implant comprenant :
un élément standard de mesure (41), formant une région de détection (411) au moyen d'un matériau radio-opaque et ayant une unité de positionnement (412) sur un côté de la région de détection (411), la région de détection (411) étant munie d'au moins un ensemble d'échelles de mesure (411a) ; et au moins un élément encerclé (42), formant un corps principal d'encerclement (421) fait d'un matériau déformable élastiquement, la région centrale du corps principal d'encerclement (421) comportant un espace emmanché (422), la taille de l'espace emmanché (422) étant changée élastiquement lorsque l'appareil orthodontique (3) est monté et reçu dans l'espace emmanché (422) du corps principal d'encerclement (421), le corps principal d'encerclement (421) comprenant en outre une unité de liaison (423), un moyen de fixation étant prévu entre l'unité de positionnement (412) et l'unité de liaison (423) de telle sorte que ladite unité de positionnement (412) et ladite unité de liaison (423) sont aptes à être engagées l'une avec l'autre.

2. Dispositif de positionnement d'implant orthodontique selon la revendication 1, dans lequel le corps principal d'encerclement (421) est utilisé pour encercler la périphérie externe de deux unités orthodontiques adjacentes (31) de l'appareil orthodontique (3) .

3. Dispositif de positionnement d'implant orthodontique selon la revendication 1, dans lequel l'unité de positionnement (412) comprend une plaque de positionnement (412a), la plaque de positionnement (412a) comprend au moins un trou traversant de positionnement (412b), et l'unité de liaison (423) comprend au moins un bloc d'arrêt (423a) qui correspond à l'au moins un trou traversant de positionnement (412b).

4. Dispositif de positionnement d'implant orthodontique adapté pour être fixé à un appareil orthodontique (3) d'un patient et utilisé entre une machine de projection de rayons X (51) et un film radiologique (52), le dispositif de positionnement d'implant comprenant :
un élément standard de mesure (41), formant une région de détection (411) au moyen d'un matériau radio-opaque et ayant une unité de positionnement (412) sur un côté de la région de détection (411), la région de détection (411) étant munie d'au moins un ensemble d'échelles de mesure (411a) ;
un élément de fixation (43), ayant une unité de liaison (43a), un moyen de fixation étant prévu entre l'unité de positionnement (412) et l'unité de liaison (43a) de telle sorte que ladite unité de positionnement (412) et ladite unité de liaison (423) sont aptes à être engagées l'une avec l'autre ; et
une pluralité d'éléments encerclés (44), reliés respectivement aux deux côtés de l'élément de fixation (43) et formant un corps principal d'encerclement (44a) fait d'un matériau déformable élastiquement, la taille d'un espace emmanché (44b) de chaque élément encerclé (44) étant apte à changer en raison de la déformation élastique de l'élément encerclé (44), lorsque l'appareil orthodontique (3) est monté et reçu dans l'espace emmanché (44b) du corps principal d'encerclement (421).

5. Dispositif de positionnement d'implant orthodontique selon la revendication 4, dans lequel au moins une partie fente (43c) est formée sur la surface et à un côté de l'élément de fixation (43).

6. Dispositif de positionnement d'implant orthodontique selon la revendication 4, dans lequel la pluralité d'éléments encerclés (44) sont agencés en parallèle et de manière symétrique par rapport aux deux côtés opposés de l'élément de fixation (43).

7. Dispositif de positionnement d'implant orthodontique selon la revendication 6, dans lequel deux corps principaux d'encerclement de la pluralité d'éléments encerclés (44) sont utilisés pour encercler respectivement les périphéries externes de deux unités orthodontiques adjacentes (31) de l'appareil orthodontique (3).

8. Dispositif de positionnement d'implant orthodontique selon la revendication 4, dans lequel l'unité de positionnement (412) comprend une plaque de positionnement (412a), la plaque de positionnement (412a) comprend au moins un trou traversant de positionnement (412b), et l'unité de liaison (43a) comprend la pluralité de blocs d'arrêt (43b) qui correspondent à la pluralité de trous traversants de positionnement (412b).

9. Dispositif de positionnement d'implant orthodontique adapté pour être fixé à un appareil orthodontique (3) d'un patient et utilisé entre une machine de projection de rayons X (51) et un film radiologique (52), le dispositif de positionnement d'implant comprenant :
un élément standard de mesure (41), formant une région de détection (411) au moyen d'un matériau radio-opaque et ayant une unité de positionnement (412) sur un côté de la région de détection (411), la région de détection (411) étant munie d'au moins un ensemble d'échelles de mesure (411a) ; et
un élément encerclé (42), intégré à un côté de l'élément standard de mesure et formant un corps principal d'encerclement (421) qui est apte à être relié à l'appareil orthodontique (3), un espace emmanché (422) étant formé collectivement par le corps principal d'encerclement (421) et l'élément standard de mesure (41), le corps principal d'encerclement étant élastiquement étendu lorsque l'appareil orthodontique (3) est monté et reçu dans l'espace emmanché (422) du corps principal d'encerclement (421) .

10. Dispositif de positionnement d'implant pour appareil orthodontique selon la revendication 9, dans lequel au moins une unité orthodontique de l'appareil orthodontique (3) est fixée dans l'espace emmanché (422) du corps principal d'encerclement (421).

11. Dispositif de positionnement d'implant pour appareil orthodontique selon la revendication 9, dans lequel le corps principal d'encerclement (421) est fixé dans une rainure centrale d'au moins une unité orthodontique de l'appareil orthodontique (3).
